(19) Europäisches Patentamt
European Patent Office
Office européen des brevets

(11) Veröffentlichungsnummer: **0 176 033**
**A2**

(12) # EUROPÄISCHE PATENTANMELDUNG

(21) Anmeldenummer: 85111836.4

(22) Anmeldetag: 19.09.85

(51) Int. Cl.[4]: C 07 D 261/18
C 07 D 261/08, C 07 D 413/04
A 61 K 31/41

(30) Priorität: 22.09.84 DE 3434947

(43) Veröffentlichungstag der Anmeldung: 02.04.86 Patentblatt 86/14

(84) Benannte Vertragsstaaten: AT BE CH DE FR GB IT LI NL SE

(71) Anmelder: BASF Aktiengesellschaft
Carl-Bosch-Strasse 38
D-6700 Ludwigshafen(DE)

(72) Erfinder: Frickel, Fritz-Frieder, Dr.
Silvanerweg 7
D-6705 Deidesheim(DE)

(72) Erfinder: Nuerrenbach, Axel, Dr.
Koenigsberger Strasse 7
D-6718 Gruenstadt(DE)

(72) Erfinder: Theobald, Hans, Dr.
Queichstrasse 6
D-6703 Limburgerhof(DE)

(54) **Isoxazolcarbonsäure-Derivate, ihre Herstellung und Verwendung.**

(57) Es wurden Isoxazolcarbonsäure-Derivate der Formel I

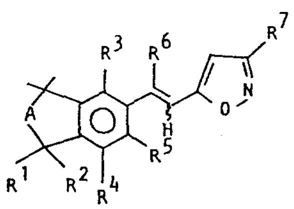

(I),

in der A und $R^1$ - $R^7$ die in der Beschreibung angebene Bedeutung besitzen, beschrieben. Die Verbindungen eignen sich als Wirkstoffe für Arzneimittel.

EP 0 176 033 A2

Isoxazolcarbonsäure-Derivate, ihre Herstellung und Verwendung

Die Erfindung betrifft neue Isoxazolcarbonsäure-Derivate, Verfahren zu deren Herstellung sowie deren Verwendung bei der Bekämpfung von Krankheiten.

Aus der EP-OS 98 591 ergibt sich, daß mit einem Thiophenring substituierte Vinyltetrahydronaphthaline rodentizide Eigenschaften besitzen.

Es wurde nun gefunden, daß Isoxazolcarbonsäure-Derivate der Formel I

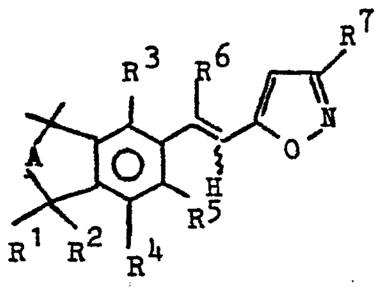 (I),

in der
$R^1$ und $R^2$ Wasserstoffatome oder Methylgruppen
$R^3$ und $R^4$ Wasserstoffatome, $C_{1-4}$-Alkylgruppen oder Methoxygruppen
$R^5$ ein Wasserstoff- oder Halogenatom oder eine $C_{1-4}$-Alkly- oder $C_{1-6}$-Alkoxygruppe
$R^6$ ein Wasserstoffatom oder eine $C_{1-6}$-Alkyl- oder $C_{3-6}$-Cycloalkylgruppe
A einen gegebenenfalls in $C_{1-4}$-Alklygruppen substituierten Methylen- oder Ethylenrest und
$R^7$ den Tetrazolyl- oder 2-Oxazolinrest oder eine Nitril- oder $C_{2-10}$-Ketalgruppe oder den Rest-$CH(R^8)-R^9$ oder $-CO-R^{10}$ bedeuten, worin
$R^8$ ein Wasserstoffatom oder eine $C_{1-3}$-Alkylgruppe
$R^9$ dasselbe wie $R^8$ oder einen Rest $-OR^{11}$ oder $NR^{12}R^{13}$ (mit $R^{11}$ in der Bedeutung eines Wasserstoffatoms, einer $C_{1-4}$-Alkyl-, $C_{2-20}$-Alkanoyl- oder gegebenenfalls substituierte Benzoylgruppe und mit $R^{12}$ und $R^{13}$ in der Bedeutung von Wasserstoffatomen, $C_{1-4}$-Alkyl-, $C_{2-20}$-Alkanoyl- oder gegebenenfalls substituierte Benzoylgruppen)
$R^{10}$ ein Wasserstoff- oder Halogenatom, den Azido-, Imidazol- oder einen Triazolrest, eine $C_{1-4}$-Alkylgruppe oder einen Rest $-OR^{14}$ oder $-NR^{15}R^{16}$ bedeuten (mit $R^{14}$ in der Bedeutung eines Wasserstoffatoms, eines $C_{1-8}$-Alkyl-, gegebenenfalls durch eine oder mehrere Hydroxygruppen oder eine $C_{1-4}$-Alkoxygruppe substituierten $C_{1-8}$-Alkyl-, gegebenenfalls substituierten Aryl- oder im Arylteil substituierten Aralkylgruppen, und mit $R^{15}$ und $R^{16}$ in der Bedeutung von Wasserstoffatomen, $C_{1-6}$-Alkyl-, gegebenenfalls durch eine oder mehrere Hydroxygruppen substituierten $C_{2-6}$-Alkylresten oder $R^{15}$ und $R^{16}$ zusammen mit dem Stickstoffatom, an das sie

gebunden sind, in der Bedeutung einer Aryl- oder Tetrazolylgruppe sowie gegebenenfalls deren physiologisch verträglichen Salze gute Arzneimittelwirkstoffe darstellen.

Von den Verbindungen der Formel I sind diejenigen bevorzugt, die die E-Konfiguration besitzen und in denen $R^1$ und $R^2$ Methylgruppen und A einen durch eine Methylgruppe substituierten Methylen- oder Ethylenrest bedeuten. Als Halogenatome für $R^{10}$ sind Fluor und Chlor bevorzugt. Substituierte Benzoylgruppen tragen als Substituenten vorzugsweise Halogenatome - insbesondere Fluor und Chlor - und/oder Methyl-, Methoxy- oder Nitrogruppen.

Als Arylgruppen sind Phenylgruppen bevorzugt, die mit einer Methyl-, Methoxy- oder Nitrogruppe substituiert sein können; als Aralkylgruppe ist die Benzylgruppe bevorzugt, die im Arylteil mit einer Methyl- oder Methoxygruppe oder mit Halogen substituiert sein kann. Als heterocyclische Reste kommen vorzugsweise der Pyrrolidino-, Piperidino- oder Morpholinrest in Betracht.

Typische Beispiele für erfindungsgemäße Verbindungen sind die folgenden Isoxazolverbindungen, die in Position 3 des Isoxazolringes noch mit den weiter unten aufgeführtne Resten substituiert sind.

5-[2-(1,1,3,3-Tetramethyl-2,3-dihydro-5(1H)-indenyl)-1-propenyl]isoxazol

5-[2-(1,1,2,3,3-Pentamethyl-2,3-dihydro-5(1H)-indenyl)-1-propenyl[isoxazol

5-[2-(5,5,8,8-Tetramethyl-5,6,7,8-tetrahydronaphth-2-yl)-1-propenyl]isoxazol

5-[2-(3,8,8-Trimethyl5,6,7,8-tetrahydronaphth-2-yl)-1-propenyl]isoxazol

5-[2-(3-Methoxy-5,5,8,8-tetramethyl-5,6,7,8-tetrahydronaphth-2-yl)-1-propenyl]isoxazol

5-[2-(3,5,5,8,8-Pentamethyl-5,6,7,8-tetrahydronaphth-2-yl)-1-propenyl]-isoxazol

5-[2-(3-Ethyl-5,5,8,8-tetramethyl-5,6,7,8-tetrahydronaphth-2-yl)-1-propenyl]isoxazol

5-[2-(3-Propyl-5,5,8,8-tetramethyl-5,6,7,8-tetrahydronaphth-2-yl)-1-propenyl]isoxazol 5-[2-(3-Butyl-5,5,8,8-tetramethyl-5,6,7,8-tetrahydronaphth-2-yl)-1-propenyl]isoxazol

5-[2-(3-(1-Methylethyl)-5,5,8,8-tetramethyl-5,6,7,8-tetrahydronaphth-2--yl)-1-propenyl]isoxazol

5-[2-(3-Butyl-5,5,8,8-tetramethyl-5,6,7,8-tetrahydronaphth-2-yl)1-propenyl]isoxazol

5-[2-(3-(1-Methylethyl)-5,5,8,8-tetramethyl-5,6,7,8-tetrahydronapth-2--yl)-1-propenyl]isoxazol

5-[2-(3-(2-Methylpropyl)-5,5,8,8-tetramethyl-5,6,7,8-tetrahydronaphth--2-yl)-1-propenyl]isoxazol

5-[2-(1,1,3,3,6-Pentamethyl-2,3-dihydro-5(1H)-indenyl)-1-propenyl]-isoxazol

5-[2-(6-Ethyl-1,1,3,3-tetramethyl-2,3-dihydro-5(1H)-indenyl)-1-propenyl]isoxazol

5-[2-(6-Propyl-1,1,3,3-tetramethyl-2,3-dihdro-5(1H)-indenyl)-1-propenyl]-isoxazol

5-[2-(6-Butyl-1,1,3,3-tetramethyl-2,3-dihydro-5(1H)-indenyl)-1-propenyl]isoxazol

5-[2-(6-(1-Methylethyl)-1,1,3,3-tetramethyl-2,3-dihydro-5(1H)-indenyl)--1-propenyl]isoxazol

5-[2-(6-(2-Methylpropyl)-1,1,3,3-tetramethyl-2,3-dihydro-5(1H)-indenyl)-1-propenyl]isoxazol

5-[2-(1,1,2,3,3,6-Hexamethyl-2,3-dihydro-5(1H)-indenyl)-1-propenyl]-isoxazol

5-[2-(6-Ethyl-1,1,2,3,3-pentamethyl-2,3-dihydro-5(1H)-indenyl)-1-propenyl]isoxazol

5-[2-(6-Propyl-1,1,2,3,3-pentamethyl-2,3-dihydro-5(1H)-indenyl)-1-propenyl]isoxazol

5-[2-(6-Butyl-1,1,2,3,3-pentamethyl-2,3-dihydro-5(1H)-indenyl)-1-propenyl]isoxazol

5-[2-(6-(1-Methylethyl)-1,1,2,3,3-pentamethyl-2,3-dihydro-5(1H)-indenyl)-1-propenyl]isoxazol

5-[2-(6-(2-Methylpropyl)-1,1,2,3,3-pentamethyl-2,3-dihydro-5(1H)-indenyl)-1-propenyl]isoxazol

5-[2-(3-Fluoro-5,5,8,8-tetramethyl-5,6,7,8-tetrahydronaphth-2-yl)-1-propenyl]isoxazol

5-[2-(3-Fluoro-5,5,8,8-tetramethyl-5,6,7,8-tetrahydronaphth-2-yl)-1-ethyenyl]isoxazol

5-[2-(3-Fluoro-5,5,8,8-tetramethyl-5,6,7,8-tetrahydronaphth-2-yl)-1-butenyl]isoxazol

5-[2-(3-Fluoro-5,5,8,8-tetramethyl-5,6,7,8-tetrahydronaphth-2-yl)-2-cyclopropyl-1-ethenyl]isoxazol

5-[2-(1,1,2,3,3-Pentamethyl-2,3-dihydro-5(1H)-indenyl)-1-ethenyl]isoxazol

5-[2-(1,1,2,3,3-Pentamethyl-2,3-dihydro-5(1H)-indenyl)-1-ethenyl]isoxazol

5-[2-(5,5,8,8-Tetramethyl-5,6,7,8-tetrahydronaphth-2-yl)-1-ethenyl]isoxazol

5-[2-(3,8,8-Trimethyl-5,6,7,8-tetrahydronaphth-2-yl)-1-ethenyl]isoxazol

5-[2-(3-Methoxy-5,6,7,8-tetramethyl-5,6,7,8-tetrahydronaphth-2-yl)-1-ethenyl]isoxazol

5-[2-(3,5,5,8,8-Pentamethyl-5,6,7,8-tetrahydronaphth-2-yl)-1-ethenyl]isoxazol

5-[2-(3-Ethyl-5,5,8,8-tetramethyl-5,6,7,8-tetrahydronaphth-2-yl)-1-ethenyl]isoxazol

5-[2-(3-Propyl-5,5,8,8-tetramethyl-5,6,7,8-tetrahydronaphth-2-yl)-1-ethenyl]isoxazol

5-[2-(3-Butyl-5,5,8,8-tetramethyl-5,6,7,8-tetrahydronaphth-2-yl)-1-ethenyl]isoxazol

5-[2-(3-(1-Methylpropyl)-5,5,8,8-tetramethyl5,6,7,8-tetrahydronaphth-2--yl)-1-ethenyl]isoxazol

5-[2-(1,1,3,3,6-Pentamethyl-2,3-dihydro-5(1H)-indenyl)-1-ethenyl]isoxazol

5-[2-(6-Ethyl-1,1,3,3,-tetramethyl-2,3-dihydro-5(1H)-indenyl)-1-ethenyl] isoxazol

5-[2-(6-Propyl-1,1,3,3-tetramethyl-2,3-dihydro-5(1H)-indenyl)-1-ethenyl] isoxazol

5-[2-(6-Butyl-1,1,3,3-tetramethyl-2,3-dihydro-5(1H)-indenyl)-1-ethenyl] isoxazol

5-[2-(6-(1-Methylethyl)-1,1,3,3-tetramethyl-2,3-dihydro-5(1H)-indenyl)-1--ethenyl]isoxazol

5-[2-(6-(2-Methylpropyl)-1,1,3,3-tetramethyl-2,3-dihydro-5(1H)-indenyl)--1-ethenyl]isoxazol

5-[2-(1,1,2,3,3,6-Hexamethyl-2,3-dihydro-5(1H)-indenyl)-1-ethenyl]isoxazol

5-[2-(6-Ethyl-1,1,2,3,3-pentamethyl-2,3-dihydro-5(1H)-indenyl)-1-ethenyl] isoxazol

5-[2-(6-Propyl-1,1,2,3,3-pentamethyl-2,3-dihydro-5(1H)-indenyl)-1-ethenyl ] isoxazol

5-[2-(6-Butyl-1,1,2,3,3-pentamethyl-2,3-dihydro-5(1H)-indenyl)-1-ethenyl]isoxazol

5-[2-(6-(1-Methylethyl)-1,1,2,3,3-pentamethyl-2,3-dihydro-5(1H)-indenyl)-1-ethenyl]isoxazol

5-[2-(6-(2-Methylpropyl)-1,1,2,3,3-pentamethyl-2,3-dihydro-5(1H)-indenyl-1-ethenyl]isoxazol

5-[2-(1,1,3,3-Tetramethyl-2,3-dihydro-5(1H)-idenyl)-1-butenyl]isoxazol

5-[2-(1,1,2,3,3-Pentamethyl-2,3-dihydro-5(1H)-idenyl)-1-butenyl]isoxazol

5-[2-(5,5,8,8-Tetramethyl-5,6,7,8-tetrahydronaphth-2-yl)-1-butenyl]isoxazol

5-[2-(3,8,8-Trimethyl-5,6,7,8-tetrahydronaphth-2-yl)-1-butenyl]isoxazol

5-[2-(3-Methoxy-5,5,8,8-tetramethyl-5,6,7,8-tetrahydronaphth-2-yl)-1-butenyl]isoxazol

5-[2-(3,5,5,8,8-Pentamethyl-5,6,7,8-tetrahydronaphth-2-yl)-1-butenyl]isoxazol

5-[2-(3-Propyl-5,5,8,8-tetramethyl-5,6,7,8-tetrahydronaphth-2-yl)-1-butenyl]isoxazol

5-[2-(3-Butyl-5,5,8,8-tetramethyl-5,6,7,8-tetrahydronaphth-2-yl)-1-butenyl]isoxazol

5-[2-(3-(1-Methylethyl)-5,5,8,8-tetramethyl-5,6,7,8-tetrahydronaphth-2-yl)-1-butenyl]isoxazol

5-[2-(3-(2-Methylpropyl)-5,5,8,8-tetramethyl-5,6,7,8-tetrahydronaphth-2-yl)-1-butenyl]isoxazol

5-[2-(1,1,3,3,6-Pentamethyl-2,3-dihydro-5(1H)-indenyl)-1-butenyl]isoxazol

5-[2-(6-Ethyl-1,1,3,3-tetramethyl-2,3-dihydro-5(1H)-indenyl)-1-butenyl]isoxazol

5-[2-(6-Propyl-1,1,3,3-tetramethyl-2,3-dihydro-5(1H)-indenyl)-1-butenyl]isoxazol

5-[2-(6-Butyl-1,1,3,3-tetramethyl-2,3-dihydro-5(1H)-indenyl)-1-butenyl]isoxazol

5-[2-(6-1-Methylethyl)-1,1,3,3-tetramethyl-2,3-dihydro-5(1H)-indenyl)-1-butenyl]isoxazol

5-[2-(6-(2-Methylpropyl)-1,1,3,3-tetramethyl-2,3-dihydro-5(1H)-indenyl)-1-butenyl]isoxazol

5-[2-(6-(2-Methylpropyl)-1,1,2,3,3-pentamethyl-2,3-dihydro-5(1H)-indenyl)-1-butenyl]isoxazol

5-[2-(6-(1-Methylethyl)-1,1,2,3,3-pentamethyl-2,3-dihydro-5(1H)-indenyl)--1-butenyl]isoxazol

5-[2-(6-Butyl-1,1,2,3,3-pentamethyl-2,3-dihydro-5(1H)-indenyl)-1-butenyl]isoxazol

5-[2-(6-Propyl-1,1,2,3,3-pentamethyl-2,3-dihydro-5(1H)-indenyl)-1-butenyl]isoxazol

5-[2-(6-Ethyl-1,1,2,3,3-pentamethyl-2,3-dihydro-5(1H)-indenyl)-1-butenyl]isoxazol

5-[2-(1,1,2,3,3,6-Hexamethyl-2,3-dihydro-5(1H)-indenyl)-1-propenyl]isoxazol

5-[2-(1,1,3,3-Tetramethyl-2,3-dihydro-5(1H)-indenyl)-2-cyclopropyl)-1--ethenyl]isoxazol

5-[2-1,1,2,3,3-Pentamethyl-2,3-dihydro-5(1H)-indenyl)-2-(cyclopropyl)--1-ethenyl]isoxazol

5-[2-(5,5,8,8-Tetramethyl-5,6,7,8-tetrahydronaphth-2-yl)-2-(cyclopropyl)-1-ethenyl]isoxazol

5-[2-(3,8,8-Trimethyl-5,6,7,8-tetrahydronaphth-2-yl)-2-(cyclopropyl)-1--ethenyl]isoxazol

5-[2-(3-Methoxy-5,5,8,8-tetramethyl-5,6,7,8-tetrahydronaphth-2-yl)-2--(cyclopropyl)-1-ethenyl]isoxazol

5-[2-(3,5,5,8,8-Pentamethyl-5,6,7,8-tetrahydronaphth-2-yl)-2-(cyclopropyl)-1-ethenyl]isoxazol

5-[2-(3-Ethyl-5,5,8,8-tetramethyl-5,6,7,8-tetrahydronaphth-2-yl)-2-(cyclopropyl)-1-ethyl]isoxazol

5-[2-(3-Propyl-5,5,8,8-tetramethyl-5,6,7,8-tetrahydronaphth-2-yl)-2--(cyclopropyl)-1-ethenyl]isoxazol

5-[2-(3-Butyl-5,5,8,8-tetramethyl-5,6,7,8-tetrahydronaphth-2-yl)-2-(cyclopropyl)-1-ethenyl]isoxazol

5-[2-(3-(1-Methylethyl)-5,5,8,8-tetramethyl-5,6,7,8-tetrahydronaphth-2-yl)-2-(cyclopropyl)-1-ethenyl]isoxazol

5-[2-(3-(2-Methylpropyl)-5,5,8,8-tetramethyl-5,6,7,8-tetrahydronaphth-2-yl)-2-(cyclopropyl)-1-ethenyl]isoxazol

5-[2-(1,1,3,3,6-Pentamethyl-2,3-dihydro-5(1H)-indenyl)-2-(cyclopropyl)-1-ethenyl]isoxazol

5-[2-(6-Ethyl-1,1,3,3-tetramethyl-2,3-dihydro-5(1H)-indenyl)-2-(cyclopropyl)-1-ethenyl]isoxazol

5-[2-(6-Propyl-1,1,3,3-tetramethyl-2,3-dihydro-5(1H)-idenyl)-2-(cyclopropyl)-1-ethenyl]isoxazol

5-[2-(6-Butyl-1,1,3,3-tetramethyl-2,3-dihydro-5(1H)-indenyl)-2-(cyclopropyl)-1-ethenyl]isoxazol

5-[2-(6-(1-Methylethyl)-1,1,3,3-tetramethyl-2,3-dihydro-5(1H)-indenyl)-2-(cyclopropyl)-1-ethenyl]isoxazol

5-[2-(6-(2-Methylpropyl)-1,1,3,3-tetramethyl-2,3-dihydro-5(1H)-indenyl)-2-(cyclopropyl)-1-ethenyl]isoxazol

5-[2-(6-(2-Methylpropyl)-1,1,2,3,3-pentamethyl-2,3-dihydro-5(1H)-indenyl)-2-(cyclopropyl)-1-ethenyl]isoxazol

5-[2-(6-(1-Methylethyl)-1,1,2,3,3-pentamethyl-2,3-dihydro-5(1H)-indenyl)-2-(cyclopropyl)-1-ethenyl]isoxazol

5-[2-(6-Butyl-1,1,2,3,3-pentamethyl-2,3-dihydro-5(1H)-indenyl)-2-(cyclopropyl)-1-ethenyl]isoxazol

5-[2-(6-Propyl-1,1,2,3,3-pentamethyl-2,3-dihydro-5(1H)-indenyl)-2-(cyclopropyl)-1-ethenyl]isoxazol

5-[2-(6-Ethyl-1,1,2,3,3,3-pentamethyl-2,3-dihydro-5(1H)-indenyl)-2-(cyclopropyl)-1-ethenyl]isoxazol

5-[2-(1,1,2,3,3,6-Hexamethyl-2,3-dihydro-5(1H)-indenyl)-2-(cyclopropyl)-1-ethenyl]isoxazol

5-[2-(1,1,3,3-Tetramethyl-2,3-dihydro-5(1H)-indenyl)-3-methyl-buten-1-yl]isoxazol

5-[2-(1,1,2,3,3-Pentamethyl-2,3-dihydro-5(1H)-indenyl)-3-methyl-buten-1-yl]isoxazol

5-[2-(5,5,8,8-Tetramethyl-5,6,7,8-tetrahydronaphth-2-yl)-3-methyl-buten-1-yl]isoxazol

5-[2-(3,8,8-Trimethyl-5,6,7,8-tetrahydronaphth-2-yl)-3-methyl-buten-1-yl]isoxazol

5-[2-(3-Methoxy-5,5,8,8-tetramethyl-5,6,7,8-tetrahydronaphth-2-yl)-3-methyl-buten-1-yl]isoxazol

5-[2-(3-Fluoro-5,5,8,8-tetramethyl-5,6,7,8-tetrahydronaphth-2-yl)-3-methyl-buten-1-yl]isoxazol

5-[2-(3,5,5,8,8-Pentamethyl-5,6,7,8-tetrahydronaphth-2-yl)-3-methyl-buten-1-yl]isoxazol

5-[2-(3-Ethyl-5,5,8,8-tetramethyl-5,6,7,8-tetrahydronaphth-2-yl)-3-methyl-buten-1-yl]isoxazol

5-[2-(3-Propyl-5,5,8,8-tetramethyl-5,6,7,8-tetrahydronaphth-2-yl)-3-methyl-buten-1-yl]isoxazol

5-[2-(3-Butyl-5,5,8,8-tetramethyl-5,6,7,8-tetrahydronaphth-2-yl)-3-methyl-buten-1-yl]isoxazol

5-[2-(3-(1-Methylethyl)-5,5,8,8-tetramethyl-5,6,7,8-tetrahydronaphth-2-yl)-3-methyl-buten-1-yl]isoxazol

5-[2-(3-(2-Methylpropyl)-5,5,8,8-tetramethyl-5,6,7,8-tetrahydronaphth-2-yl)-3-methyl-buten-1yl]isoxazol

5-[2-(1,1,3,3,6-Pentamethyl-2,3-dihydro-5(1H)-indenyl)-3-methyl-buten-1-yl]isoxazol

5-[2-(6-Ethyl-1,1,3,3-tetramethyl-2,3-dihydro-5(1H)-indenyl)-3-methyl-buten-1-yl]isoxazol

5-[2-(6-Propyl-1,1,3,3-tetramethyl-2,3-dihydro-5(1H)-indenyl)-3-methyl-buten-1-yl]isoxazol

5-[2-(6-Butyl-1,1,3,3-tetramethyl-2,3-dihydro-5(1H)-indenyl)-3-methyl-buten-1-yl]isoxazol.

5-[2-(6-(1-Methylethyl)-1,1,3,3-tetramethyl-2,3-dihydro-5(1H)-indenyl)-3-methyl-buten-1-yl]isoxazol

5-[2-(6-(2-Methylpropyl)-1,1,3,3-tetramethyl-2,3-dihydro-5(1H)-indenyl)-3-methyl-buten-1-yl]isoxazol

5-[2-(6-(2-Methylpropyl)-1,1,2,3,3-pentamethyl-2,3-dihydro-5(1H)-indenyl)-3-methyl-buten-1-yl]isoxazol

5-[2-(6-(1-Methylethyl)-1,1,2,3,3-pentamethyl-2,3-dihydro-5(1H)-indenyl)-3-methyl-buten-1-yl]isoxazol

5-[2-(6-Butyl-1,1,2,3,3-pentamethyl-2,3-dihydro-5(1H)-indenyl)-3-methyl-buten-1-yl]isoxazol

5-[2-(6-Propyl-1,1,2,3,3-pentamethyl-2,3-dihydro-5(1H)-indenyl)-3-methyl-buten-1-yl]isoxazol

5-[2-(6-Ethyl-1,1,2,3,3-pentamethyl-2,3-dihydro-5(1H)-indenyl)-3-methyl-buten-1-yl]isoxazol

5-[2-(1,1,2,3,3,6-Hexamethyl-2,3-dihydro-5(1H)-indenyl)-3-methyl-buten-1-yl]isoxazol

5-[2-(5,5,8,8-Tetramethyl-1-methoxy-4-methyl-5,6,7,8-tetrahydro-naphth-2-yl)-1-ethenyl]isoxazol

Diese Verbindungen sind in Position 3 des Isoxazolrings mit den folgenden typischen Resten substituiert:

3-Carboxy-, 3-Methoxycarbonyl-, 3-Ethoxycarbonyl-, 3-Propyloxycarbonyl-, 3-(1-Methyl)ethoxycarbonyl-, 3-butoxycarbonyl-, 3-Octyloxycarbonyl-, 3-(2-Methoxy)ethoxycarbonyl-, 3-(2-Hydroxy)-ethoxycarbonyl-, 3-(2,3-Dihydroxy)propoxycarbonyl,3-Benzyloxycarbonyl-, 3-Cyano-, 3-Formyl-, 3-Hydroxymethyl-, 3-Methyl-, 3-Ethyl-, 3-Propyl-, 3-Chlorformyl-, 3-Fluorformyl-, 3-Azidoformyl-, 3-Acetyl-, 3-Methoxymethyl-, 3-Ethoxymethyl-, 3-Propyloxymethyl-, 3-Butyloxymethyl-, 3-Benzyloxymethyl-; 3-Acetoxymethyl-, 3-Propionyloxymethyl-, 3-Hexadecanoyloxymethyl-, 3-Aminomethyl-, 3-Benzoyloxymethyl-, 3-(3,4-Dimethoxy)benzoyloxymethyl-, 3-Methylaminomethyl-, 3-Ethylaminomethyl-, 3-Butylaminomethyl-, 3-Propylaminomethyl-,

3-Acetyl-, 3-Benzoylaminomethyl-, 3-(4-Methoxy)benzoylaminomethyl-, 3--Acetylaminomethyl-, 3-Propionylaminomethyl-, 3-Palmitoylaminomethyl-, 3-N-Imidazolyl)-carbonyl-, 3-(N-Triazolyl)-carbonyl-.

Die erfindungsgemäßen Verbindungen kann man auf verschiedenen an sich bekannten Wegen herstellen. Z. B. kann man eine Carbonylverbindung der Formel (II)

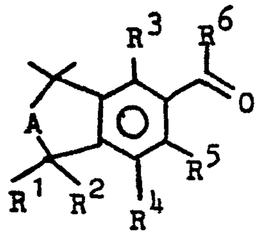 (II),

in der A, $R^1$, $R^2$, $R^3$, $R^4$, $R^5$ und $R^6$ die oben angegebenen Bedeutungen haben, mit einer Phosphorverbindung der Formel (III)

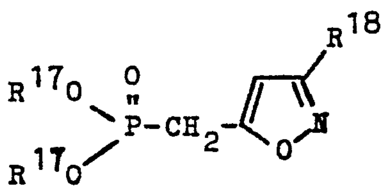 (III),

in der $R^{17}$ eine $C_{1-3}$-Alkylgruppe bedeutet und $R^{18}$ für eine $C_{1-7}$-Alkylgruppe eine Carbo-$C_{1-8}$-alkoxygruppe, oder für eine $C_{1-4}$-Alkoxymethyl--$C_{1-4}$-alkoxymethylgruppe nach der sog. Wittig-Horner-Reaktion umsetzen. Zweckmäßigerweise arbeitet man in einem Lösungsmittel in Gegenwart der für Wittig-Horner-Reaktionen üblichen basischen Verbindungen.

Die Umsetzung nach Wittig-Horner verläuft bei einer Temperatur von bis zu 100 °C, zweckmäßig bei 20 bis 50 °C. Die Umsetzung kann bei atmosphärischem Druck oder in einem geschlossenen Gefäß unter erhöhtem Druck, gegebenenfalls unter Erwärmung auf den angegebenen Temperaturbereich durchgeführt werden.

Man kann diese Umsetzung in Gegenwart eines Verdünnungs- oder Lösungsmittels, beispielsweise eines niederen gesättigten Dialkylethers, Dialkylglykolethers oder cyclischen Ethers, wie Diethylether, Ethyl-tert.--butylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Isooctan, eines niederen aliphatischen Ketons, wie Aceton, Methylethylketon oder Methylisobutylketon, eines Dialkylformamids, wie Dimethyl- oder Diethylformamid, oder in Mischungen der genannten Lösungsmittel durchführen. Bevorzugt verwendet man cyclische Ether, wie Dioxan oder Tetrahydrofuran sowie insbesondere Dimethylformamid oder deren Mischungen, wobei die Umsetzung im allgemeinen bei einer Temperatur bis zu 30 °C abläuft.

Die Umsetzungen werden in Gegenwart eines Deprotonierungsmittels für das Phosphat (III) vorgenommen. Geeignet sind Alkalimetallhydride und Alkalimetallamide, insbesondere des Natriums und Kaliums, die Natrium- und Kaliumsalze von Dimethylsulfoxid, Alkyllithiumverbindungen wie n--Butyllithium oder Alkalimetallalkoholate, vorzugsweise Natriummethanolat und Natriumethanolat.

Die Isoxazolsäureester der Formel (I), in denen $R^7$ eine Carbo-$C_{1-8}$-alkoxygruppe bedeutet, werden, falls dies erwünscht ist, in die freien Carbonsäuren durch Esterverseifung überführt. Umgekehrt läßt sich die freie Säure in bekannter Weise verestern.

Die Hydrolyse läuft in üblicher Weise bei im allgemeinen bis zu 120 °C ab und wird vorzugsweise bei Raumtemperatur durchgeführt.

Sie kann bei atmosphärischem Druck oder in einem geschlossenen Gefäß unter erhöhtem Druck durchgeführt werden.

Zweckmäßigerweise wird die Verseifung bzw. Veresterung in Gegenwart eines Verdünnungs- oder Lösungsmittels, beispielsweise eines Dialkylglykolethers oder cyclischen Ethers, wie 1,2-Dimethoxyethan, Tetrahydrofuran oder Dioxan, eines niederen aliphatischen Ketons, wie Aceton, Methylethylketon oder Methylisobutylketon, die Veresterung insbesondere in dem vorgesehenen niederen aliphatischen Alkohol, wie Methanol, Ethanol, Propanol oder Isopropanol, gegebenenfalls in Gegenwart von Wasser bzw. in Mischungen der genannten Lösungsmittel mit Wasser durchgeführt.

Bevorzugte Lösungsmittel sind wäßrige Mischungen von Ethanol und Methanol, wobei die Umsetzung beim Siedepunkt des Reaktionsgemisches durchgeführt wird.

Die Verseifung geschieht bevorzugt in Gegenwart von Alkali, wie Alkalimetallhydroxide, Alkalimetallcarbonate und -hydrogencarbonate, insbesondere des Natriums und Kaliums, organischen tertiären Basen, wie Pyridin oder niedere Trialkylamine, wie Trimethyl- oder Triethylamin in Gemischen mit Wasser. Dabei wird die verwendete Base im Verhältnis zum Ester in stöchiometrischer Menge oder in geringem Überschuß eingesetzt. Vorzugsweise wird Natrium- oder Kaliumhydroxid verwendet.

Die Veresterung geschieht vorteilhaft durch Einleiten von Chlorwasserstoffgas in das alkoholische Reaktionsgemisch. Die Methylester können auch durch Einwirkung von Diazomethan auf die freie Säure erhalten werden.

Carbonsäuren der allgemeinen Formel (I), in der dann $R^7$ für COOH steht, kann man in reaktionsfähige Säure-Derivate der Formel IV überführen

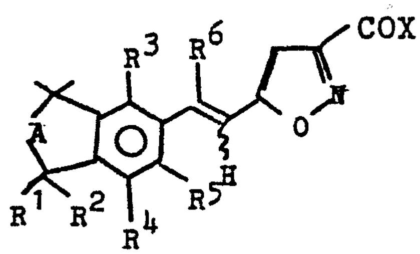

(IV),

in der A, $R^1$, $R^2$, $R^3$, $R^4$, $R^5$, $R^6$ und n die oben angegebenen Bedeutungen haben und X für einen üblichen, reaktionsfähigen Rest eines gemischten Säureanhydrids oder ein Halogen steht. Diese werden zweckmäßigerweise in einem Lösungsmittel und gegebenenfalls in Gegenwart eines säurebindenden Mittels mit Aminen $HN<^{R^{12}}_{R^{13}}$ oder Alkoholen $HOR^{11}$ zu Amiden und Estern umsetzt. Die Bezeichnung X in der Formel (IV) stellt ein Halogenatom, insbesondere Chlor oder auch Brom oder z. B. den N-Oxysuccinimid-Rest dar. Diese Umsetzungen von IV verlaufen bei einer Temperatur von bis zu 50 °C bei Atmosphärendruck oder in einem geschlossenen Gefäß unter erhöhtem Druck.

Man kann diese Umsetzungen in Gegenwart eines Verdünnungs- oder Lösungsmittels, beispielsweise eines niederen gesättigten Dialkylethers, Dialkylglykolethers oder cyclischen Ethers, wie Diethylether, Ethyl-tert.-butylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Isooctan, eines niederen aliphatischen Ketons, wie Aceton, Methylethylketon oder Methylisobutylketon, eines Dialkylformamids, wie Dimethyl- oder Diethylformamid, oder in Mischungen der genannten Lösungsmittel durchführen. Bevorzugt verwendet man lineare oder cyclische Ether, wie Diethylethter oder Tetrahydrofuran sowie insbesondere Dimethylformamid, wobei die Umsetzung im allgemeinen bei einer Temperatur bis zu 30 °C abläuft.

Üblicherweise wird die Umsetzung in Gegenwart einer Base als säurebindendem Mittel vorgenommen. Geeignete Basen sind Alkalimetallcarbonate, -hydrogencarbonate, insbesondere des Natriums und Kaliums, organische tertiäre Basen, wie Pyridin oder niedere Trialkylamine, wie Trimethyl-oder Triethylamin. Dabei wird die verwendete Base im Verhältnis zum eingesetzten Isoxazolsäurehalogenid in stöchiometrischer Menge oder in geringem Überschuß verwendet.

Eine andere Möglichkeit zur Herstellung der erfindungsgemäßen Verbindungen geht von entsprechenden Säuren der Formel IV aus, wobei in diesem Fall X für OH steht; man setzt in Gegenwart eines die Carboxylgruppe aktivierenden, wasserabspaltenden Mittels in einem Lösungsmittel mit Aminen $HN{<}^{R^{15}}_{R^{16}}$ um.

Als wasserabspaltende aktivierende Reagentien können die bei der Peptidsynthese üblichen Reagentien verwendet werden, wie sie beispielsweise von Schröder und Lübke in "The Peptides", Band I, Academic Press, N. Y., 1965, Seiten 77 bis 128, beschrieben werden. Das allgemeine Prinzip der Umsetzung besteht in der Aktivierung der Carboxylgruppe, beispielsweise durch Behandlung mit einem Carbodiimid, wie N,N'-Dicyclohexylcarbodiimid, oder durch intermediäre Bildung des Säureazids, eines gemischten Anhydrids (beispielsweise mit Kohlensäuremonoestern), eines aktivierten Esters (beispielsweise des p-Nitrophenylesters) oder eines heterocyclischen Amids (beispielsweise eines Imidazolids) der entsprechenden Carbonsäure (IV).

Die Behandlung einer an der Carboxylgruppe aktivierten Verbindung mit Aminen $HN{<}^{R^{15}}_{R^{16}}$ führt dann zu den erfindungsgemäßen Verbindungen. Die Aktivierungs- und Verknüpfungsreaktionen können in Lösungsmitteln, vorzugsweise in N,N-Dimethylformamid, Tetrahydrofuran, Dioxan, Methylenchlorid, Nitromethan, Acetonitril, Dimethylsulfoxid, N,N-Dimethylacetamid und Hexamethylphosphorsäuretriamid vorgenommen werden. Eine geeignete Temperatur für beide Stufen, d. h. der Reaktion der Säure mit dem Kupplungsmittel und die Reaktion des aktivierten Zwischenproduktes mit Aminen $HN{<}^{R^{15}}_{R^{16}}$ liegt z. B. zwischen 20 und 100 °C. Man kann dabei entweder stufenweise vorgehen, indem man das aktivierte Zwischenprodukt vor der Zugabe des Amins isoliert und vorteilhaft, indem man die Reaktionspartner nacheinander ohne Isolierung von Zwischenstufen zur Reaktion bringt. Bei einer bevorzugten Verkünpfungsmethode verwendet man das N,N-Carbonyldiimidazol und arbeitet in Dimethylformamid, wobei die Reaktionstemperatur für beide Stufen bei 20 bis 60 °C liegt.

Zur Herstellung von erfindungsgemäßen Amiden, in denen $R^{15}$ für Wasserstoff steht, ist die direkte Aminolyse von Estern (mit dem Rest $-OR^{14}$) mit primären Aminen $H_2N-R^{15}$ bei Raumtemperatur ohne Lösungsmittel oder gegebenenfalls in Gegenwart eines organischen Lösungsmittels, wie Alkoholen, Ethern, Dialkylformamiden oder Mischungen dieser Lösungsmittel bevorzugt.

Ein Carbonsäurehalogenid der Formel (IV), vorzugsweise das Säurechlorid, kann durch Reaktion mit 2-Aminoethanol oder 2-Amino-2-methylpropanol-1 und anschließende Cyclisierung in ein Oxazolinderivat der Formel (I) überführt werden.

Eine Carbonsäure, ein Carbonsäureester oder ein Carbonsäureamid der Formel (I) kann in an sich bekannter Weise zu den Alkoholen bzw. Aminen der Formel (I) reduziert werden. Vorteilhaft wird die Reduktion mit Hilfe eines Metallhydrids oder Alkalimetallhydrids in Gegenwart eines geeigneten Lösungsmittels durchgeführt. Als Metallhydride werden vorzugsweise komplexe Metallhydride wie Lithiumaluminiumhydrid oder Diisobutylaluminiumhydrid eingesetzt. Als Lösungsmittel werden beim Arbeiten mit Lithiumaluminiumhydrid Ether eingesetzt, wie Diethylether, Dioxan oder Tetrahydrofuran. Führt man die Reduktion aber mit Diisobutylaluminiumhydrid oder einem Alkoxynatriumaluminiumhydrid durch, so ist die Verwendung von Kohlenwasserstoffen wie Hexan oder Toluol bevorzugt.

Ein Amin oder ein Alkohol der Formel (I) kann in an sich bekannter Weise mit einem Alkanoylhalogenid oder -anhydrid, einem Aralkylhalogenid oder -anhydrid oder einem Aroyl- oder Heteroaroylhalogenid oder -anhydrid zweckmäßigerweise in einem inerten Verdünnungs- oder Lösungsmittel, z. B. einem niederen aliphatischen Keton wie Aceton, Methylethylketon oder Methylisobutylketon, einem Dialkylformamid wie Dimethylformamid oder Diethylformamid oder mit überschüssigem Acylierungsmittel als Verdünnungs- oder Lösungsmittel in die erfindungsmäßen Amide und Ester der Formel (I) überführt werden. Bevorzugt werden die Umsetzungen in Gegenwart einer Base als säurebindendes Mittel in einem zwischen -20 °C und dem Siedepunkt des Reaktionsgemisches liegenden Temperaturbereich vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin, oder niedere Trialkylamine, wie Trimethyl- oder Triethylamin. Dabei können die Basen im Verhältnis zum eingesetzten Alkylierungsmittel in katalytischer Menge oder in stöchiometrischer Menge bzw. in geringem Überschuß verwendet werden.

Ein Alkohol der Formel (I) kann mit Alkylhalogeniden $R^{11}$-J, $R^{11}$-Br oder $R^{11}$-Cl in Gegenwart von Alkalimetallhydriden, vorzugsweise Natriumhydrid oder in Gegenwart von Alkyllithium-Verbindungen, vorzugsweise n-Butyllithium, in einem organischen Lösungsmittel wie Tetrahydrofuran, Dioxan, 1,2-Dimethoxyethan, Methyl-tert.-butylether oder bei Verwendung von Natriumhydrid auch in Dimethylformamid in einem zwischen -10 °C und 40 °C liegenden Temperaturbereich zu einem Ether der Formel (I) umgesetzt werden.

Ein Alkohol der Formel (I) kann mit geeigneten Oxidationsmitteln, vorzugsweise Mangan(IV)-oxid, gegebenenfalls auf einem anorganischen Trägermaterial wie Silicagel oder Aluminiumoxid zu einem Aldehyd der Formel (I) oxidiert werden. Vorteilhaft arbeitet man in einem inerten organischen Lösungsmittel, beispielsweise einem Kohlenwasserstoff wie Hexan oder in einem Ether wie beispielsweise Tetrahydrofuran oder in Mischungen der genannten Lösungs- und Verdünnungsmittel im Temperaturbereich zwischen -10 °C und 30 °C. Die benötigte Reaktionszeit ist im wesentlichen von der Oxidationsaktivität des eingesetzten Mangan(IV)-oxids abhängig.

Die erfindungsgemäßen Verbindung der Formel (I) können einheitlich die cis- oder trans-Struktur haben oder Mischungen der beiden E-/Z-Isomere darstellen. Eine Isomerisierung bei den vorerwähnten Umsetzungen kann außerdem nich ausgeschlossen werden. Die in solchen Fällen z.B. resultierende Mischung der erfindungsgemäßen Verbindung der Formel (I) kann durch HPLC-Analyse oder durch ein $^{13}$CNMR-Spektrum quantitativ bestimmt und das jeweils gewünschte Isomere gegebenenfalls durch fraktionierende Kristallisation oder Chromatographie mit beispielsweise Kieselgelsäulen oder durch präparative HPL-Chromatographie isomerenrein isoliert werden.

Einige der erfindungsgemäßen Verbindungen weisen ein acides Wasserstoffatom auf und können daher mit Basen in üblicher Weise in ein physiologisch verträgliches, gut wasserlösliches Salz überführt werden. Geeignete Salze sind beispielsweise Ammonium-, Alkalimetall-, insbesondere des Natriums, Kaliums und Lithiums, Erdalkalimetallsalze, insbesondere des Calciums oder Magnesiums sowie Salze mit geeigneten organischen Basen, wie mit niederen Alkylaminen, z.B. Methylamin oder Ethylamin, mit substituierten niederen Alkylaminen, insbesondere hydroxysubstituierten Alkylaminen, wie Diethanolamin, Triethanolamin oder Tris-(hydroxymethyl)-aminomethan, Piperidin oder Morpholin.

Erfindungsgemäß erhaltene Amine der Formel (I) lassen sich nach bekannter Verfahrensweise in ihre Säureadditionssalze mit physiologisch verträglichen Säuren überführen. Als übliche physiologisch verträgliche organische oder anorganische Säure kommen beispielsweise in Betracht Salzsäure, Bromwasserstoffsäure, Phosphorsäure oder Schwefelsäure und als organische Säure beispielsweise Oxalsäure, Maleinsäure, Fumarsäure, Milchsäure, Weinsäure, Äpfelsäure, Zitronensäure, Salicylsäure, Adipinsäure oder Benzoesäure oder können aus Fortschritten der Arzneimittelforschung Band <u>10</u>, Seiten 224 - 225, Birkhäuser Verlag, Basel und Stuttgart, 1966, entnommen werden.

Die erfindungsgemäßen Verbindungen und ihre physiologisch verträglichen Salze können aufgrund ihrer pharmakologischen Eigenschaften bei der topischen und systemischen Therapie und auch Prophylaxe von Praekanzerosen und Karzinomen der Haut, der Schleimhäute und innerer Organe sowie bei der topischen und systemischen Therapie von Akne, Psoriasis und anderer mit pathologisch veränderter Verhornung einhergehenden dermatologischen Erkrankungen sowie zur Behandlung von rheumatischen Erkrankungen, insbesondere solcher entzündlicher oder degenerativer Art, die Gelenke, Muskeln, Sehnen und andere Teile des Bewegungsapparates befallen, verwendet werden. Ein bevorzugtes Indikationsgebiet ist neben der Therapie von dermatologischen Erkrankungen die prophylaktische und therapeutische Behandlung von Praekanzerosen und Tumoren.

Die pharmakologischen Wirkungen können beispielsweise in den nachfolgenden Testmodellen aufgezeigt werden: Die erfindungsgemäßen Verbindungen heben an Hamstertreachealgewebe in vitro die nach Vitamin-A-Mangel einsetzende Keratinisierung auf. Diese Keratinisierung gehört zur Frühphase der Carcinogenese, die in einer ähnlichen Technik in vivo nach der Initiation durch chemische Verbindungen, durch energetische Strahlung oder nach viraler Zelltransformation durch die erfindungsgemäßen Verbindungen der Formel (I) inhibiert wird. Diese Methodik kann aus Cancer Res. 36, 964 - 972 (1976) oder aus Nature 250, 64 - 66 (1974) und Nature 253, 47 - 50 (1975) entnommen werden.

Darüber hinaus werden durch die erfindungsgemäßen Verbindungen die Proliferationsraten bestimmter maligne veränderter Zellen inhibiert. Diese Methodik kann aus J. Natl. Cancer Inst. 60, 1035 - 1041 (1978), Experimental Cell Research 117, 15 - 22 (1978) und Proc. Natl. Acad. Sci. USA 77, 2936 - 2940 (1980) entnommen werden.

Die antiarthritische Wirkung der erfindungsgemäßen Verbindungen kann in üblicher Weise im Tierexperiment im Adjuvans-Arthritis-Modell bestimmt werden. Die dermatologische Aktivität, beispielsweise für die Behandlung von Akne, kann u. a. durch die komedolytische Aktivität und die Fähigkeit nachgewiesen werden, die Anzahl der Zysten im Modell der Rhino--Maus zu reduzieren.

Diese Methode ist von L. H. Kligman et al in The Journal of Investigative Dermatology 73, 354 - 358 (1979) und von J.A. Mezick et al. in "Models of Dermatology" (Ed. Maibach, Löwe), Vol. 2, S. 59-63, Karger, Basel 1985) beschrieben.

Die Testsubstanz wurde in einem geeigneten Vehikel auf der gesamten Rückenpartie der Rhino-Maus topisch aufgetragen (100 µl), einmal täglich, an fünf aufeinanderfolgenden Tagen pro Woche, zwei Wochen lang ungefähr 72 h nach der letzten Behandlung wurde die dorsale Haut entfernt und in 0,5 %iger Essigsäure 18 h bei 4-6°C belassen. Danach wurde eine ca. 2 x 5 $cm^2$ große Fläche herausgeschnitten, die Epidermis abgeschält, auf einen Objektträger aufgebracht (mit der dermalen Seite nach oben) und mittels Alkohol/Xylol wasserfrei gespült, bis die Epidermis durchsichtig erscheint. Die Probe wurde durch Überziehen mit Permount fixiert und mikroskopisch ausgewertet. Gemessen wurde der Durchmesser von jeweils 10 Utriculi in jeweils 5 frei gewählten Feldern und daraus durch Vergleich mit der unbehandelten Kontrollgruppe die durchschnittliche Reduktion des Utriculusdurchmessers berechnet. Die folgende Tabelle zeigt die erhaltenen Ergebnisse.

Tabelle

| Substanz | Dosis mg/ml | Reduktion des Utriculusdurchmessers in % |
|---|---|---|
| Beispiel 17a | 2 | 75,6 |
| | 0,2 | 45,1 |

Dementsprechend sind ein weiterer Gegenstand der Erfindung therapeutische Mittel zur topischen und systemischen Anwendung, die eine Verbindung der Formel (I) neben üblichen Trägerstoffen oder Verdünnungsmitteln als Wirkstoff enthalten, und die Verwendung einer Verbindung der Formel (I) zur Herstellung eines Arzneimittels.

Die Herstellung der therapeutischen Mittel oder Zubereitungen erfolgt mit den üblichen flüssigen oder festen Trägerstoffen oder Verdünnungsmitteln und den in üblicher Weise verwendeten pharmazeutisch-technischen Hilfsstoffen, entsprechend der gewünschten Applikationsart und mit einer zur Anwendung geeigneten Dosierung, in üblicher Weise beispielsweise durch Vermischen des Wirkstoffs mit den an sich in solchen Präparaten üblichen festen oder flüssigen Träger- und Hilfsstoffen.

Die Mittel können dementsprechend peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,001 bis 1 %iger Konzentration, bevorzugt in 0,001 bis 0,1 %iger Konzentration und bei systemischer Anwendung vorzugsweise in einer Einzeldosis von 0,1 bis 50 mg enthalten und täglich in einer oder mehreren Dosierungen je nach Art und Schwere der Erkrankungen verabreicht werden.

Üblicherweise verwendete pharmazeutische technische Hilfsstoffe sind beispielsweise für die lokale Anwendung Alkohole, wie Isopropanol, oxethyliertes Ricinusöl oder oxethyliertes hydriertes Ricinusöl, Polyacrylsäure, Glycerinmonostearat, Paraffinöl, Vaseline, Wollfett, Polyethylenglykol 400, Polyethylenglykol 400-Stearat sowie ethoxylierter Fettalkohol, für die systemische Anwendung Milchzucker, Propylenglykol und Ethanol, Stärke, Talk, Polyvinylpyrrolidon. Den Präparaten kann gegebenenfalls ein Antioxidationsmittel, beispielsweise Tocopherol sowie butyliertes Hydroxyanisol oder butyliertes Hydroxytoluol oder geschmacksverbessernde Zusätze, Stabilisierungsmittel, Emulgiermittel, Gleitmittel usw. zugesetzt werden. Voraussetzung ist, daß alle bei der Herstellung pharmazeutische Zubereitung verwendeten Stoffe toxikologisch unbedenklich sind und mit den verwendeten Wirkstoffen verträglich sind.

Herstellung der erfindungsgemäßen Verbindungen

<u>Beispiel 1</u>

(E)-5-[2-(1,1,2,3,3-Pentamethyl-2,3-dihydro-5(1H)-indenyl)-1-propenyl]isoxazol-3-carbonsäureethylester

Zu einer Suspension von 300 ml Tetrahydrofuran und 9 g 80 %iges Natriumhydrid, das zuvor mit Petrolether vom 20 %igen Paraffin-Anteil befreit worden war, wurde innerhalb von 30 min. eine Lösung von 87,4 g Diethyl(3--carboethoxyisoxazolyl-5)methylenphosphonat in 200 ml Tetrahydrofuran bei ca. 40°C zugetropft. Anschließend wurde noch 15 Min. nachgerührt und innerhalb von 10 min. eine Lösung von 50,6 g 5-Acetyl-1,1,2,3,3-pentamethylindan in 200 ml Tetrahydrofuran zugetropft. Nach 2 h wurde der Ansatz auf 2 l Eis-Wasser gegossen und dreimal mit Diethylether extrahiert. Die Extrakte wurden mit Wasser neutral gewaschen, über Natriumsulfat getrocknet und am Rotationsverdampfer von flüchtigen Bestandteilen befreit. Der entstehende Feststoff wurde mit 100 ml Methanol digeriert. Es verblieben nach dem Trocknen in einer Stickstoffatmosphäre 31,8 g (E)-5-[2-(1,1,2,3,3-Pentamethyl-2,3-dihydro-5(1H)-indenyl)-1-propenyl]-isoxazol-3-carbonsäureethylester vom Schmp. 90 - 91°C.

Das $^1H$-NMR-Spektrum belegt, daß es sich um die isomerenreine, trans-konfigurierte Titelverbindung handelt.

Beispiel 2

(E)-5-[2-(1,1,2,3,3-Pentamethyl-2,3-dihydro-5(1H)-indenyl)-1-propenyl] isoxazol-3-carbonsäure

6 g (E)-5-[2-(1,1,2,3,3-Pentamethyl-2,3-dihydro-5(1H)-indenyl)-1-propenyl]isoxazol-3-carbonsäureethylester wurden mit 1,2 g Kaliumhydroxid in einem Gemisch aus 400 ml EThanol und 20 ml Wasser 6 h bei Raumtemperatur belassen. Nach dem Überführen der gesamten Reaktionsmasse in 1 Liter Wasser wurde mit 2 N Salzsäure angesäuert, der erhaltene Niederschlag abfiltriert und mit 100 ml Methanol digeriert. Nach erneutem Absaugen des Kristallisats und Trocknen im Stickstoffstrom verbleiben 3,8 g (E)-5-[2--(1,1,2,3,3-Pentamethyl-2,3-dihydro-5(1H)-indenyl)-1-propenyl]isoxazol-3--carbonsäure, vom Schmp. 168 - 169°C. Das $^1H$-NMR-Spektrum belegt, daß es sich um die isomerenreine trans-konfigurierte Titelverbindung handelt was auch die reversed-phase HPLC-Analyse (RP-C18; 150 bar; Ethanol/20 % Wasser) bestätigt.

Beispiel 3

(E)-5-[2-(1,1,2,3,3-Pentamethyl-2,3-dihydro-5(1H)-indenyl)-1-propenyl] isoxazol-3-carbonsäureamid

4,5 g (E)-5-[2-(1,1,2,3,3-Pentamethyl-2,3-dihydro-5(1H)-indenyl)-1-propenyl]isoxazol-3-carbonsäureethylester wurden mit 150 ml konzentrierter Ammoniaklösung in 200 ml Ethanol 10 h gerührt. Nach dem Überführen der gesamten Reaktionsmasse in 500 ml Wasser wurde der erhaltene Niederschlag abfiltriert und mit wenig eiskaltem Methanol nachgewaschen. Nach dem Trocknen verblieben 3,6 g (E)-5-[2-(1,1,2,3,3-Pentamethyl-2,3-dihydro-5(1H)-indenyl)-1-propenyl]isoxazol-3-carbonsäureamid, Schmp. 196 bis 197°C.

Beispiel 4

(E)-5-[2-(1,1,2,3,3-Pentamethyl-2,3-dihydro-5(1H)-indenyl)-1-propenyl]--N-(2,3-dihydroxypropyl)-isoxazol-3-carbonsäureamid

3,7 g (E)-5-[2-(1,1,2,3,3-Pentamethyl-2,3-dihydro-5(1H)-indenyl)-1-propenyl]isoxazol-3-carbonsäureethylester wurden mit 25 ml 1-Amino-2,3--propandiol in 70 ml Ethanol 10 h belassen. Nach dem Überführen der

gesamten Reaktionsmasse in 500 ml Wasser wurde mit 2 N Salzsäure angesäuert. Der erhaltene Niederschlag wird abfiltriert und mit wenig Methanol nachgewaschen. Nach dem Trocknen im Stickstoffstrom verblieben 2,1 g (E)-5-[2-(1,1,2,3,3-Pentamethyl-2,3-dihydro-5(1H)-indenyl)-1-propenyl]-N-(2,3-dihydroxypropyl)-isoxazol-3-carbonsäureamid Schmp. 150 - 151 °C.

Beispiel 4 a

Analog Beispiel 4 wurde aus (E)-5-[2-(1,1,2,3,3-Pentamethyl-2,3-dihydro-5(1H)-indenyl)-1-propenyl]isoxazol-3-carbonsäureethylester und Ethanolamin-(E)-5-[2-(1,1,2,3,3-Pentamethyl-2,3-dihydro-5(1H)-indenyl)-1-propenyl]-N-(2-hydroxyethyl)-isoxazol-3-carbonsäureamid erhalten, Schmp. 143 - 144°C.

Die in der nachfolgenden Tabelle angegebenen Verbindungen wurden gemäß Beispiel 1 hergestellt.

| Beispiel Nr. | A | $R^1$ | $R^2$ | $R^3$ | $R^4$ | $R^5$ | $R^6$ | $R^7$ | Konfig. der Doppelbindung |
|---|---|---|---|---|---|---|---|---|---|
| 5 | $-CH_2-CH_2-$ | $CH_3$ | $CH_3$ | H | H | $CH_3$ | $CH_3$ | $-COOC_2H_5$ | 5 |
| 6 | $-CH_2-CH_2-$ | $CH_3$ | $CH_3$ | H | H | $C_2H_5$ | $CH_3$ | $-COOC_2H_5$ | E |
| 7 | $-CH_2-CH_2-$ | $CH_3$ | $CH_3$ | H | H | $C_3H_7$ | $CH_3$ | $-COOC_2H_5$ | E |
| 8 | $-CH_2-CH_2-$ | $CH_3$ | $CH_3$ | H | H | 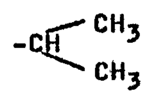 | $CH_3$ | $-COOC_2H_5$ | E |
| 9 | $-CH_2-CH_2-$ | $CH_3$ | $CH_3$ | H | H | 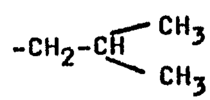 | $CH_3$ | $-COOC_2H_5$ | E |
| 10 | $-CH_2-CH_2-$ | $CH_3$ | $CH_3$ | H | H | $CH_3$ | H | $-COOC_2H_5$ | E |

0176033

| Beispiel Nr. | A | $R^1$ | $R^2$ | $R^3$ | $R^4$ | $R^5$ | $R^6$ | $R^7$ | Konfig. der Doppelbindung |
|---|---|---|---|---|---|---|---|---|---|
| 11 | $-CH_2-CH_2-$ | $CH_3$ | $CH_3$ | H | H | $OCH_3$ | $CH_3$ | $-COOC_2H_5$ | E |
| 12 | $-CH_2-CH_2-$ | $CH_3$ | $CH_3$ | H | H | $OCH_3$ | H | $-COOC_2H_5$ | E |
| 13 | $H_2C-CH$ | $CH_3$ | $CH_3$ | H | H | H | 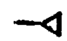 | $-COOC_2H_5$ | E |
| 14 | $H_3-CH$ | $CH_3$ | $CH_3$ | H | H | H | $C_3H_7$ | $-COOC_2H_5$ | E |
| 14a | $-CH_2-CH_2-$ | $CH_3$ | $CH_3$ | H | H | H | $CH_3$ | $-COOC_2H_5$ | E |
| 14b | $-CH_2-CH_2-$ | $CH_3$ | $CH_3$ | H | H | F | H | $-COOC_2H_5$ | E |

| Schmp. [°C] | Name |
|---|---|
| 104 - 105 | (E)-5-[2-(3,5,5,8,8-Pentamethyl-5,6,7,8-tetrahydronaphth-2-yl)-1-propenyl]isoxazol-3-carbonsäureethylester |
| 97 - 98 | (E)-5-[2-(3-Ethyl-5,5,8,8-tetramethyl-5,6,7,8-tetrahydro-naphth-2-yl)-1-propenyl]isoxazol-3-carbonsäureethylester |
| 118 - 120 | (E)-5-[2-(3-Propyl-5,5,8,8-tetramethyl-5,6,7,8-tetrahydro-naphth-2-yl)-1-propenyl]isoxazol-3-carbonsäureethylester |
| 127 - 128 | (E)-5-[2-[3-(1-Methyl)ethyl-5,5,8,8-tetramethyl-5,6,7,8-tetrahydro-naphth-2-yl]-1-propenyl]isoxazol-3-carbonsäureester |
| 107 - 108 | (E)-5-[2-[3-(2-methyl)propyl-5,5,8,8-tetramethyl-5,6,7,8-tetrahydro-naphth-2-yl]-1-propenyl]isoxazol-3-carbonsäureethylester |
| 96 - 97 | (E)-5-[2-(3,5,5,8,8-Pentamethyl-5,6,7,8-tetrahydro-naphth-2-yl)-ethenyl]isoxazol-3-carbonsäureethylester |
| 86 - 88 | (E)-5-[2-(3-Methoxy-5,5,8,8-tetramethyl-5,6,7,8-tetrahydro-naphth-2-yl)-1-propenyl]isoxazol-3-carbonsäureethylester |
| 102 - 103 | (E)-5-[2-(3-Methoxy-5,5,8,8-tetramethyl-5,6,7,8-tetrahydro-naphth-2-yl)-ethenyl]isoxazol-3-carbonsäureethylester |
| 85 - 86 | (E)-5-[2-(1,1,2,3,3-Pentamethyl-2,3-dihydro-5(1H)-indenyl)-2-(cyclopropyl)-1-propenyl]isoxazol-3-carbonsäureethylester |
| | (E)-5-[2-(1,1,2,3,3-Pentamethyl-2,3-dihydro-5(1H)-indenyl)-1-pentenyl]isoxazol-3-carbonsäureethylester |
| 78 - 79 | (E)-5-[2-(5,5,8,8-Tetramethyl-5,6,7,8-tetrahydro-naphth-2-yl)-1-propenyl]isoxazol-3-carbonsäureethylester |
| 118 - 119 | (E)-5-[2-(5,5,8,8-Tetramethyl-5,6,7,8-tetrahydro-naphth-2-yl)-1-propenyl]isoxazol-3-carbonsäureethylester |

Die in der nachfolgenden Tabelle aufgeführten Verbindungen werden gemäß Beispiel 2 hergestellt.

| Beispiel Nr. | A | $R^1$ | $R^2$ | $R^3$ | $R^4$ | $R^5$ | $R^6$ | $R^7$ | Konfig. der Doppelbindung |
|---|---|---|---|---|---|---|---|---|---|
| 15 | $-CH_2-CH_2-$ | $CH_3$ | $CH_3$ | H | H | $OCH_3$ | $CH_3$ | $COOC_2H_5$ | 5 |
| 16 | $-CH_2-CH_2-$ | $CH_3$ | $CH_3$ | H | H | $OCH_3$ | H | COOH | E |
| 17 | $H_3C-CH$ | $CH_3$ | $CH_3$ | H | H | H | 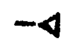 | COOH | E |
| 17a | $-CH_2-CH_2-$ | $CH_3$ | $CH_3$ | H | H | H | $CH_3$ | $COOH_2H_5$ | E |
| 17b | $-CH_2-CH_2-$ | $CH_3$ | $CH_3$ | H | H | F | $CH_3$ | COOH | E |

| Schmp. [°C] | Name |
|---|---|
| | (E)-5-[2-(3-Methoxy-5,5,8,8-tetramethyl-5,6,7,8-tetrahydro-naphth-2-yl)-1-propenyl]isoxazol-3-carbonsäure |
| 166 - 167 | (E)-5-[2-(3-Methoxy-5,5,8,8-tetramethyl-5,6,7,8-tetrahydro-naphth-2-yl)-ethenyl]isoxazol-3-carbonsäure |
| 159 - 160 | (E)-5-[2-(1,1,2,3,3-Pentamethyl-2,3-dihydro-5(1H)-indenyl)-2-(cyclopropyl)-1-propenyl]isoxazol-3-carbonyäure |
| 174 - 175 | (E)-5-[2-(5,5,8,8-Tetramethyl-5,6,7,8-tetrahydro-naphth-2-yl)-1-propenyl]isoxazol-3-carbonsäure |
| 184 - 185 | (E)-5-[2-(3-Fluoro-5,5,8,8-tetramethyl-5,6,7,8-tetrahydro-naphth-2-yl)-1-propenyl]isoxazol-3-carbonsäure |

Beispiel 18

Analog zu der in Beispiel 1 beschriebenen Arbeitsweise wurde aus 5-Acetyl-1,1,2,3,3-pentamethylindan und Diethyl(3-methylisoxazolyl-5-)methylenphosphonat(E)-3-Methyl-5-[2-(1,1,2,3,3-pentamethyl-2,3-dihydro-5(1H)-indenyl)-1-propenyl]isoxazol erhalten, Schmp. 120 - 121°C.

In gleicher Weise wurden die nachfolgenden Substanzen synthetisiert.

| Bsp. Nr. | A | $R^1$ | $R^2$ | $R^3$ | $R^4$ | $R^5$ | $R^6$ | $R^7$ | Konfig. der Doppelbindung | Schmp. [°C] | Name |
|---|---|---|---|---|---|---|---|---|---|---|---|
| 19 | $-CH_2-CH_2-$ | $CH_3$ | $CH_3$ | H | H | H | $CH_3$ | $CH_3$ | E | 118-119 | (E)-3-Methyl-5-[2-(5,5,8,8--tetramethyl-5,6,7,8-tetra-hydro-napth-2-yl)-1-propenyl]isoxazol |
| 20 | $-CH_2-CH_2-$ | $CH_3$ | $CH_3$ | H | H | $CH_3$ | $CH_3$ | $CH_3$ | E | 107-108 | (E)-3-Methyl-5-[2-(3,5,5,8,8-penta-methyl-5,6,7,8-tetrahydro-naphth-2-yl)-1-propenyl] isoxazol |
| 21 | $-CH_2-CH_2-$ | $CH_3$ | $CH_3$ | H | H | $CH_3$ | $CH_3$ | $CH_3$ | E | 128-129 | (E)-3-Methyl-5-[2-(3-methoxy--5,5,8,8-tetramethyl-5,6,7,8-tetrahydronapth-2-yl)-1-propenyl]isazol |

Beispiel 22

Analog zu der in Beispiel 1 beschriebenen Arbeitsweise wurde aus 6-Acetyl-1,1,4,4-tetramethyltetralin und Diethyl(3-methoxymethylisoxazolyl-5)methylenphosphonat (E)-3-Methoxymethyl-5-[2-(5,5,8,8-tetramethyl-5,6,7,8-tetrahydronaphth-2-yl)-1-propenyl]isoxazol, Schmp. 72 - 73°C, erhalten.

Beispiel 23

(E)-3-Hydroxymethyl-5-[2-(5,5,8,8-tetramethyl-5,6,7,8-tetrahydro-naphth-2-yl)-1-propenyl]isoxazol

Zu einer gerührten Suspension von 1 g Lithiumaluminiumhydrid in 150 ml Diethylether tropfte man eine Lösung von 5 g (E)-5-[2-(5,5,8,8-Tetramethyl-5,6,7,8-tetrahydro-naphth-2-yl)-1-propenyl]isoxazol-3-carbonsäureethylester in 50 ml Diethylether. Nach 3 h versetzte man tropfenweise mit 200 ml einer 10 %igen wäßrigen Weinsäurelösung und extrahiert mehrmals mit Diethylether. Die vereinigten organischen Phasen wurden neutral gewaschen und unter wasserfreiem Natriumsulfat getrocknet. Der nach dem Eindampfen verbleibende kristalline Rückstand wurde aus 25 ml Methanol umkristallisiert. Man erhielt so 2,3 g der Titelverbindung, Schmp. 138 - 139°C.

Beispiel 24

Analog zu der in Beispiel 23 beschriebenen Arbeitsweise wurde aus (E)-5-[2-(1,1,2,3,3-Pentamethyl-2,3-dihydro-5(1H)-indenyl)-1-propenyl]isoxazol-3-carbonsäureethylester (E)-3-Hydroxymethyl-5-[2-(1,1,2,3,3-pentamethyl-2,3-dihydro-5(1H)-indenyl)-1-propenyl]isoxazol erhalten.

Beispiel 25

(E)-3-Acetoxymethyl-5-[2-(1,1,2,3,3-pentamethyl-2,3-dihydro-5(1H)-indenyl)-1-propenyl]isoxazol

Eine Lösung von 4,9 g (E)-3-Hydroxymethyl-5-[2-(1,1,2,3,3-pentamethyl-2,3-dihydro-5(1H)-indenyl)-1-propenyl]isoxazol in 25 ml Diethylether und 10 ml Pyridin wurde tropfenweise bei 0°C bis 5°C mit 4,7 ml Acetylchlorid versetzt. Anschließend rührte man noch 3 h bei Raumtemperatur; man goß auf Wasser und extrahierte mit Diethylether. Die organische Phasen wurden mit 1 N Salzsäure behandelt, mit Wasser neutral gewaschen und über Natriumsulfat getrocknet. Der nach dem Eindampfen der Lösung verbleibende Feststoff wurde aus 20 ml Ethanol umkristallisiert. So erhielt man 5,2 g der Titelverbindung, Schmp. 91 - 92°C.

Beispiel 26

(E)-3-[N-(3-Hydroxy-4-carboxyphenylamino)thioxomethylcarbamoyl]-5-[2--(1,1,2,3,3-pentamethyl-2,3-dihydro-5(1H)-indenyl)-1-propenyl]isoxazol

In eine Lösung aus 5 g (E)-5-[2-(1,1,2,3,3-Pentamethyl-2,3-dihydro-5(1H)--indenyl)-1-propenyl]isoxazol-3-carbonsäure, 30 ml Tetrahydrofuran und 1,4 ml Pyridin wurden 1,2 ml Thionylchlorid getropft. Nach 45 min. bei Raumtemperatur gab man zu diesem Gemisch 1,6 g Kaliumrhodanid in 15 ml Aceton und nach weiteren 45 min. 2,8 4-Amino-2-hydroxybenzoesäure in 30 ml Tetrahydrofuran.

Nach 8 h wurde die gesamte Reaktionsmasse in eine Lösung aus 400 ml Methanol und 400 ml Wasser gegossen. Den erhaltenen Feststoff filtriert man ab und wisch ihn auf der Nutsche nacheinander mit Methanol und n-Heptan. Nach dem Trocknen im Stickstoffstrom verblieben 4,3 g der Titelverbindung, Schmp. 221 - 222°C.

0176033

Patentansprüche

1. Isoxazolcarbonsäure-Derivate der Formel I

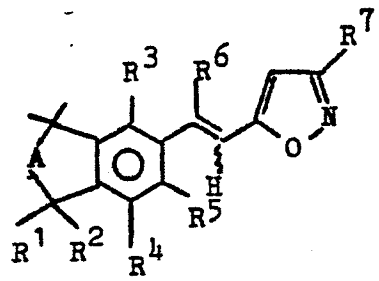

(I),

in der

$R^1$ und $R^2$ Wasserstoffatome oder Methylgruppen

$R^3$ und $R^4$ Wasserstoffatome, $C_{1-4}$-Alkylgruppen oder Methoxygruppen

$R^5$ ein Wasserstoff- oder Halogenatom oder eine $C_{1-4}$-Alkyl- oder $C_{1-6}$--Alkoxygruppe

$R^6$ ein Wasserstoffatom oder eine $C_{1-6}$-Alkyl- oder $C_{3-6}$-Cycloalkylgruppe

A einen gegebenenfalls mit $C_{1-4}$-Alkylgruppen substituierten Methylen- oder Ethylenrest und

$R^7$ den 2-Oxazolinrest oder den Rest $-CH(R^8)-R^9$ oder $-CO-R^{10}$ bedeuten, worin

$R^8$ ein Wasserstoffatom oder eine $C_{1-3}$-Alkylgruppe

$R^9$ dasselbe wie $R^8$ oder einen Rest $-OR^{11}$ oder $NR^{12}R^{13}$ (mit $R^{11}$ in der Bedeutung eines Wasserstoffatoms, einer $C_{1-4}$-Alkyl-, $C_{2-20}$-Alkanoyl- oder gegebenenfalls substituierte Benzoylgruppe und mit $R^{12}$ und $R^{13}$ in der Bedeutung von Wasserstoffatomen, $C_{1-4}$-Alkyl-, $C_{2-20}$-Alkanoyl- oder gegegbenenfalls substituierter Benzoylgruppen)

$R^{10}$ ein Wasserstoff- oder Halogenatom, den Azido-, Imidazol- oder einen Triazolrest, eine $C_{1-4}$-Alkylgruppe oder einen Rest $-OR^{14}$ oder $-NR^{15}R^{16}$ bedeuten (mit $R^{14}$ in der Bedeutung eines Wasserstoffatoms, einer $C_{1-8}$-Alkyl-, gegebenenfalls durch eine oder mehrere hydroxygruppen oder eine $C_{1-4}$-Alkoxygruppe substituierten $C_{1-8}$-Alkyl-, gegebenenfalls substituierten Aryl- oder im Arylteil substituierten Aralkylgruppe, und mit $R^{15}$ und $R^{16}$ in der Bedeutung von Wasserstoffatomen, $C_{1-6}$-Alkyl-, gegebenenfalls durch eine oder mehrere Hydroxygruppen substituierten $C_{2-6}$-Alkylresten oder $R^{15}$ und $R^{16}$ zusammen mit den Stickstoffatomen, an das sie gebunden sind, in der Bedeutung eines heterocyclischen Restes

sowie gegebenenfalls deren physiologisch verträglichen Salze.

2. (E)-5-[2-(3-Fluoro-5,5,8,8-tetramethyl-5,6,7,8-tetrahydronaphth-2-yl)-1-propenyl]isoxazol-3-carbonsäure.

3. Isoxazolcarbonsäure-Derivate gemäß Anspruch 1 zur Verwendung bei der Bekämpfung von Krankheiten.

Patentansprüche

1. Verfahren zur Herstellung der Isoxazolcarbonsäure-Derivate der Formel I

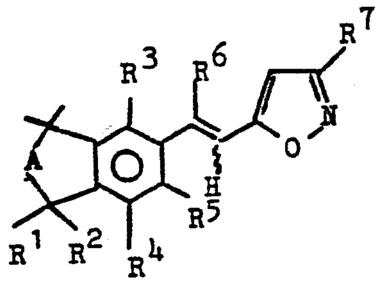

(I),

in der

$R^1$ und $R^2$ Wasserstoffatome oder Methylgruppen

$R^3$ und $R^4$ Wasserstoffatome, $C_{1-4}$-Alkylgruppen oder Methoxygruppen

$R^5$ ein Wasserstoff- oder Halogenatom oder eine $C_{1-4}$-Alkyl- oder $C_{1-6}$-Alkoxygruppe

$R^6$ ein Wasserstoffatom oder eine $C_{1-6}$-Alkyl- oder $C_{3-6}$-Cycloalkylgruppe

A einen gegebenenfalls mit $C_{1-4}$-Alkylgruppen substituierten Methylen- oder Ethylenrest und

$R^7$ den 2-Oxazolinrest oder den Rest $-CH(R^8)-R^9$ oder $-CO-R^{10}$ bedeuten, worin

$R^8$ ein Wasserstoffatom oder eine $C_{1-3}$-Alkylgruppe

$R^9$ dasselbe wie $R^8$ oder einen Rest $-OR^{11}$ oder $NR^{12}R^{13}$ (mit $R^{11}$ in der Bedeutung eines Wasserstoffatoms, einer $C_{1-4}$-Alkyl-, $C_{2-20}$-Alkanoyl- oder gegebenenfalls substituierte Benzoylgruppe und mit $R^{12}$ und $R^{13}$ in der Bedeutung von Wasserstoffatomen, $C_{1-4}$-Alkyl-, $C_{2-20}$-Alkanoyl- oder gegegbenenfalls substituierter Benzoylgruppen)

$R^{10}$ ein Wasserstoff- oder Halogenatom, den Azido-, Imidazol- oder einen Triazolrest, eine $C_{1-4}$-Alkylgruppe oder einen Rest $-OR^{14}$ oder $-NR^{15}R^{16}$ bedeuten (mit $R^{14}$ in der Bedeutung eines Wasserstoffatoms, einer $C_{1-8}$-Alkyl-, gegebenenfalls durch eine oder mehrere hydroxygruppen oder eine $C_{1-4}$-Alkoxygruppe substituierten $C_{1-8}$-Alkyl-, gegebenenfalls substituierten Aryl- oder im Arylteil substituierten Aralkylgruppe, und mit $R^{15}$ und $R^{16}$ in der Bedeutung von Wasserstoffatomen, $C_{1-6}$-Alkyl-, gegebenenfalls durch eine oder mehrere Hydroxygruppen substituierten $C_{2-6}$-Alkylresten oder $R^{15}$ und $R^{16}$ zusammen mit den Stickstoffatomen, an das sie gebunden sind, in der Bedeutung eines heterocyclischen Restes

sowie gegebenenfalls deren physiologisch verträglichen Salze, <u>dadurch gekennzeichnet</u>, daß man eine Carbonylverbindung der Formel (II)

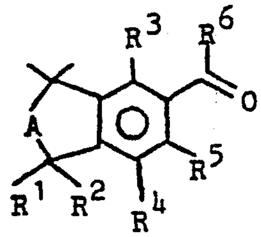 (II),

in der A, $R^1$, $R^2$, $R^3$, $R^4$, $R^5$ und $R^6$ die oben angegebenen Bedeutungen haben, mit einer Phosphorverbindung der Formel (III)

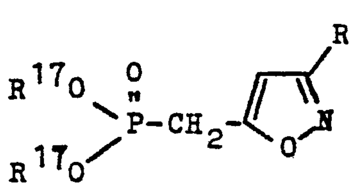 (III),

in der $R^{17}$ eine $C_{1-3}$-Alkylgruppe bedeutet und $R^{18}$ für eine $C_{1-7}$-Alkylgruppe eine Carbo-$C_{1-8}$-alkoxygruppe, oder für eine $C_1$-4-Alkoxymethyl--$C_{1-4}$-Alkoxymethylgruppe nach der sog. Wittig-Horner-Reaktion umsetzt und die so erhaltenen Verbindungen gewünschtenfalls anschließend in andere Verbindungen der Formel I überführt.

2. Verfahren gemäß Anspruch 1, dadurch gekennzeichnet, daß man (E)-5-[2-(3-Fluoro-5,5,8,8-tetramethyl-5,6,7,8-tetrahydronaphth-2-yl)-1-propenyl]isoxazol-3-carbonsäure herstellt.